# EUROPEAN PATENT SPECIFICATION

(11) **EP 3 181 542 B1**
(45) Date of publication and mention of the grant of the patent: **13.03.2019**
(21) Application number: 15382632.6
(22) Date of filing: 16.12.2015
(51) Int. Cl.: C07C 29/80, C07C 29/10, C07C 31/20

(54) **METHOD FOR THE PREPARATION OF GLYCOLS**
VERFAHREN ZUR HERSTELLUNG VON GLYCOLEN
PROCÉDÉ DE PRÉPARATION DE GLYCOLS

(43) Date of publication of application: 21.06.2017
(73) Proprietor: Repsol, S.A., 28045 Madrid (ES)
(72) Inventor: PÉREZ VALENCIA, Juan Pedro, 28935 Móstoles (ES); FERNÁNDEZ VILLAR, Félix, 28935 Móstoles (ES)
(74) Representative: Balder IP Law, S.L.

(56) References cited:
- US-A- 3 922 314
- US-B1- 6 514 388
- R. G. M. Elsinghorst: "Optimization of the reactor section of a catalytic ethyleneglycol plant", , 1 May 1994 (1994-05-01), pages 1-A108, XP055273774, Retrieved from the Internet: URL:http://repository.tudelft.nl/islandora /object/uuid:f28997ca-b592-4133-ad9d-26e56 db9f053?collection=research [retrieved on 2016-05-19]

## Description

### FIELD OF THE INVENTION

The invention relates to an improved method for the hydration of alkylene oxides in order to obtain the corresponding glycols.

### BACKGROUND OF THE INVENTION

Commercial processes for the preparation of alkylene glycols, for example, ethylene glycol or propylene glycol, involve the liquid phase hydration of the corresponding alkylene oxide. By "hydration" in the present disclosure it is understood the process wherein the alkylene oxide reacts with water in a catalytic or non-catalytic process without use of other intermediates such as carbonates. The reaction usually requires a temperature between 100°C and 200°C and it also referred to as "thermal hydration". Thus, in the present disclosure "hydration" and "thermal hydration" are considered equivalents unless otherwise indicated.

The reaction usually proceeds at a pressure sufficient to maintain reactants in the liquid phase, typically at 5-50 bar depending on the conditions (e.g. water excess, temperature). In the hydration each alkylenne oxide molecule can undergo a ring-opening reaction with a molecule of water to provide a monoalkylene glycol, or with a further molecule of alkylene glycol to form a dialkylene glycol. These competitive reactions can continue to form trialkylene glycols and higher homologues. The hydration of alkylene oxides always results in a mixture of mono-, di-, tri-alkylene glycols and higher homologues. In order to favor the formation of the monoalkylene glycol (e.g. monoethylene glycol - MEG), usually more interesting, a large molar excess of water is used (see, for example, Ullmann's Encyclopedia of Industrial Chemistry, seventh Edition, Ethylene Glycol chapter). A typical commercially practiced method for making, for example ethylene glycol, uses a water:ethylene oxide molar proportion between 4:1 and 20:1 (i.e.between about 38 wt% to about 10 wt% of ethylene oxide) to provide 80-90 wt% of monoethylene glycol (MEG), 9-15 wt% of diethylene glycol (DEG), and 1-5 wt% of triethylene glycol (TEG).

In the non-catalyzed thermal hydration processes, the larger the excess of water the greater, the yield of the mono-alkylene glycols with respect to other higher homologues such as di- or tri-alkylene glycols. This tendency however reaches a limit, and at very large excesses of water the improvement in mono-alkylene glycol proportion becomes negligible, and does not compensate the economic costs of having to add large amounts of water which have to be removed after reaction. Said post-reaction water removal (dewatering) is energy intensive, and thus costly. There is therefore a need to find a compromise in order to obtain an equilibrium between the yield of mono-alkylene and the use of water.

The resulting dry alkylene glycol mixture, is purified in its components, usually the monoalkylene glycol being the mayor product. Thus, a typical alkylene glycol plant comprises a reaction zone, a dewatering zone and a purification zone.

The standard dewatering zone is placed at the exit of the reaction zone and uses several evaporators. The most frequent scheme is the so-called triple effect. Three evaporators are arranged in series at decreasing pressures so that the vapor boiled off in one vessel can be used to heat the next due to the thermal gradient. The process is energetically integrated. Only the first evaporator requires an external source of heat. Vapor of the first and second evaporators is passed through the reboiler of second and third evaporators respectively. Vapor of the last evaporator is condensed using a external cooler. Condensed water from dewatering is sent back to the reaction zone for recycling. Multiple effect evaporation does not usually remove all the water, leaving a 2 to 10 percent weight of water in the glycol stream, and is many times followed downstream by a vacuum distillation tower that completes the drying of the glycol product. Once the glycol product has been dewatered and dried, it is typically purified in a purification zone, usually through distillation. Such arrangements are described in many documents such as, Ullmann's Encyclopedia of Industrial Chemistry, seventh Edition, Ethylene Glycol chapter or in different patent application documents such as US 2012/0203015 A1 or US 5,514,388 B1.

The reactor zone is typically made of one or more hydrating reactors, the plug adiabatic flow reactors being the ones most used. The alkylene oxide is typically fully converted to glycol. The reaction process is typically maintained at a pressure above the reaction mixture bubble point in order to assure liquid phase. The reaction is highly exothermic so that at lower water to alkylene oxide feed ratios, a series of reactors with inter-stage cooling are needed to remove heat of reaction and maintain reaction product temperature below an upper limit. In a configuration of multiple reactors, water is usually fed at the first stage, and the alkylene oxide feed is split between each reactor in equal or different portions.

US 4,822,926 discloses the treatment with a large excess of water of a residual feed comprising dilute ethylene oxide (0.5-3.0 wt%) and dilute salts (0.5-3.0 wt%), in order to transform all ethylene oxide into ethylene glycol. In this way the authors could avoid the costly treatment of ethylene oxide before disposal. After the treatment, water is removed. According to US 4,822,926, water is removed through "flashers", but a full reading of the document indicates that such flashers are the usual evaporators (triple effect) containing plates used in the conventional glycol synthesis, and not what is commonly understood as flasher. US 4,822,926 is thus concerned with hydrating all ethylene oxide in order to dispense with the later treatments required for its recovery, rather than with improving the efficiency of a thermal hydration process.

Many variations have been proposed to improve the efficiency of thermal hydration of alkylene oxides. For example, the EC/EG process of SHELL (e.g. US 2012/095271) integrating the production of ethylene oxide with its later hydration via the corresponding carbonates. Other attempts to improve the catalytic technology are disclosed in US 6,514,388. It describes a process in which at least one of the columns used in the dewatering zone are provided with a stripper.

Any modification resulting in a more efficient use of water may have a significant impact in the overall efficiency of the process.

### DESCRIPTION OF THE DRAWINGS

Figure 1: Represents an exemplary configuration of the method of the invention using one intermediate dewatering flash process.
Figure 2: Represents an examplary configuration using one intermediate dewatering flash process which does not form part of the presently claimed invention.
Figure 3: Represents an exemplary configuration of the method of the invention using three hydrating reactors and one intermediate dewatering flash process.
Figure 4: Represents an exemplary configuration of the method of the invention using three hydrating reactors and two dewatering flash process.
Figure 5: Represents a further exemplary configuration of the method of the invention using three hydrating reactors and two dewatering flash process.
Figure 6: Represents the configuration discussed in Comparative Example 1 and represents a thermal hydration using a single hydrating reactor according to prior art.
Figure 7: Represents the configuration discussed in Example 1 and represents a thermal hydration using a single hydrating reactor according to the invention.
Figure 8: Represents the configuration discussed in Comparative Example 2 and represents a thermal hydration using three hydrating reactors according to prior art.
Figure 9: Represents the configuration discussed in Example 2 and represents a thermal hydration using three hydrating reactors according to the invention.

### SUMMARY OF THE INVENTION

The inventors now provide an improved process for the preparation of alkylene glycols which makes a more efficient use of water and significantly decreases the energy requirements of the process. Contrary to previous strategies, which only undergo dewatering process once the reaction mixture has left the reaction zone, the inventors now provide a process wherein the reaction mixture undergoes one or more flash processes .

Thus, the invention refers to a method for preparing an alkylene glycol comprising hydrating with water an alkylene oxide in a hydrating reactor placed in a reaction zone, said alkylene oxide being in at least 5 wt% with respect to the total feed, characterized in that the product mixture of said hydrating reactor is subject to a dewatering flash process in a flasher, wherein said flasher is a flash separator drum and wherein the bottoms leaving the flasher are re-pressurized prior entry into the following hydrating reactor.

US 4,822,926 is only concerned with the total transformation of ethylene oxide into ethylene glycol. It is directed to currents containing only dilute amounts of glycol (about less than 3.0 wt%) and it does not suggest the use of flashers, not to mention that the overall efficiency of a hydration process can be improved by the use of flashers on feeds containing a higher concentration of glycols.

By applying these flash processes to more concentrated currents which result from thermal hydration, the inventors provide a process that surprisingly requires the use of less external water in an amount similar to the water internally recirculated. More advantages come from flashing the product of thermal hydration which were not anticipated in the prior art. For example, flashing the reaction product also cools the reaction feed towards the following reactor in the case of multiple reactors in series, thus reducing the energy required for inter-stage cooling. Another advantage of the process which was not realized in the prior art is that flashed water from the reactor product can be condensed and at the same time pre-heat external water fed to the first reactor, reducing energy consumption in the reaction, and leading to a significant improvement in heat-integration. Also, lighter than water impurities can be removed from the reaction product before entering the following reactor in a multiple reactors arrangement, reducing secondary reactions. Impurities can be removed by means of non-condensable purge or stripping from flashed water before recycling it to reaction feed.

### DETAILED DESCRIPTION OF THE INVENTION

Therefore, the method of this invention is a synergistic combination of the hydration reaction and a flash evaporation, combined to enhance the reaction's selectivity and the efficient use of water. The reaction zone comprises at least one hydrating reactor, usually between 2 and 8 reactors, and at least one flasher downstream of one of said hydrating reactors (e.g. downstream the last reactor). The configuration used more frequently is one were the reaction zone comprises 2 or 3 or 4 hydrating reactors. Therefore, the flash (or flashers) used in the invention can be installed at different points in the process downstream from at least one hydrating reactor.

### Equipment

Hydrating reactors can be of any type, and they can all be of the same type or different, and can also be operated under the same or different conditions. Many of such reactors are disclosed in the art and known to the skilled person. Reactors are typically tubular plug-flow type. Adiabatic reactors are typically used in the process of the invention because they typically provide a better use of energy, but other non-adiabatic reactors also fall within the scope of the invention. Thus, the most widely used reactors in the thermal hydration of alkylene oxides are the adiabatic tubular plug-flow type, although others are available. It is preferred that at least one of the reactors is an adiabatic plug-flow reactor, preferably that all reactors are adiabatic plug-flow reactors. Each reactor has one or more inlets and one or more outlets. The first hydrating reactor of the reaction zone may have one or more inlets for water and one or more inlets for the alkylene oxide. Different configurations can be applied for feeding.

The above are not however the only alternatives available, and the skilled person can adjust the process of the invention without adversely affecting the outcome. For example, the product mixture and the further alkylene oxide added to a second and/or subsequent hydrating reactors can be both pre-mixed. Also, if necessary, the pressure of the mixture entering a hydrating reactor can be adjusted in order to ensure that the reactants are in the liquid phase. For example, if, as a consequence of a flash process, the reaction mixture (bottoms of the flash) does not have and adequate pressure, it can be re-pressurized before entering the hydrating reactor.

According to the invention, the flasher is a flash separator drum. For example, the most advantageous units have no heating surface, and comprise a simple flash separator drum for adiabatic flash vaporization of preheated liquids, i.e. without any plates. Also within the scope of the invention are units with a heating surface, consisting of a simple flash separator drum with internal coils and/or a jacket, for flash vaporization with heat input. Separator drums can include any type of known internal configurations in order to favor liquid-vapor separation. Non-limiting examples are demisters or impingement separators. The flasher can thus include internals in order to favor liquid-vapor disengagement and off-vapor demist. Flasher are typically operated at a pressure that ranges from reaction pressure to lower than atmospheric pressure; the lower the pressure, the higher the vapor flow flashed and the lower the temperature of the resulting product and water. The pressure can be adjusted so as adapt the process to the equipment used depending on, for example, its size, configuration, composition of the feed, or composition desired at the outlet. Preferred operating pressure is 0.1 and 15 bar, for example from 2 to 8 bar so as to adjust flash vapor temperature. The conditions in each of the flashing units may be the same or may be different.

### Process

The first step of the method comprises feeding water and the alkylene oxide into the reactor. This can be done through any of the known processes described in the art. Water and the alkylene oxide can be supplied directly to the first hydration (or the only) reactor at specific flows, temperature and pressure. Alternatively, they can be pre-mixed and pre-heated, and then fed to the first hydrating reactor as a mixture.

The process disclosed herein is not limited to a particular alkylene oxide. The most frequently used are C₁-C₆ alkylene oxide, typically ethylene oxide or propylene oxide. Other akylene oxides having higher molecular weights can be used, such as butylene oxide or cyclohexylene oxide.

Typically, the water and the alkylene oxide are fed separately to the first (or the only) reactor, and water is pre-heated before entering. The same is true for other reactors in the reaction zone. The product mixture entering a second and/or subsequent hydrating reactors can come from the previous hydrating reactor(s) after being cooled by cooling means. The present invention offers the possibility of dispensing with such cooling means or greatly reducing their use since the flash process already reduces the temperature of the feed.

One of the key parameters for controlling the proportion between the different possible glycol products is the proportion between water and alkylene oxide. In the present invention, the amount of alkylene oxide is at least 5 wt% with respect to the total amount of starting materials, i.e. the sum of water and alkylene oxide (the amount of other minor components and impurities is ignored for this calculation). The alkylene oxide can be for example between 5 wt% and 40 wt%, for example between 10 wt% and 30wt%. The process typically operates under water:ethylene oxide molar proportions comprised between 2:1 and 20:1 (i.e. between about 38 wt% to about 10 wt% of in the case of ethylene oxide), for example between 4:1 to 15:1.

Operation conditions in the hydrating reactor(s) can be adjusted. Typical operation temperature is comprised between 100 to 250ºC, generally from 110°C to 220°C and more specifically from 125°C to 190°C, or from 120°C to 195°C. For security reasons and to improve efficiency, operating pressure is typically above reactor mixture bubble point in order to maintain liquid phase. The conditions in each of the hydrating reactors may be the same or may be different. The process of the invention can be applied to catalytic and to non-catalytic processes, preferably to non-catalytic processes.

In embodiments which are not part of the presently claimed invention and wherein the process involves a single reactor, the product exiting said reactor is sent to a flasher were part of the water is flashed evaporated. Typical operating conditions of the flasher are adiabatic conditions at a pressure of 1-10 bar, for example, 1-7 bar or 1-3 bar. The present invention provides the advantages disclosed herein when the method comprises more than one reactor. The most frequently installed operations use 2 to 4 reactors. Usually, the reaction zone comprises a first hydrating reactor, a second hydrating reactor and a third hydrating reactor, but the skilled person can appreciate that more reactors are possible. Flashers can be placed at the exit of any of the reactors, and their operation conditions adjusted. Typical operating conditions of the flasher are adiabatic conditions at a pressure of 1-10 bar, for example, 1-7 bar or 1-3 bar. The exact pressure of each flasher can be adjusted in order to optimize the process. For example, typical conditions include that the following flasher operates at lower pressures than the previous flasher. Following the procedures known in the art, cooling means are installed between each of the reactors in order to reduce the temperature of the feed before entering the next reactor. Such cooling means usually involve heat exchange with cooler currents of the plant. Thus, the skilled person can devise different configurations based on the teachings of the process taught herein and the advantages it provides. It is generally preferred that a flasher is placed after the last reactor, that is, between the last reactor and the dewatering zone, preferably, the crude product from said last flasher being directly sent to the dewatering zone, optionally after being used to cool the product of the first hydrating reactor. If necessary, vapor leaving a flasher can be condensed and re-pressurized prior to recirculating into the hydrating reactor.

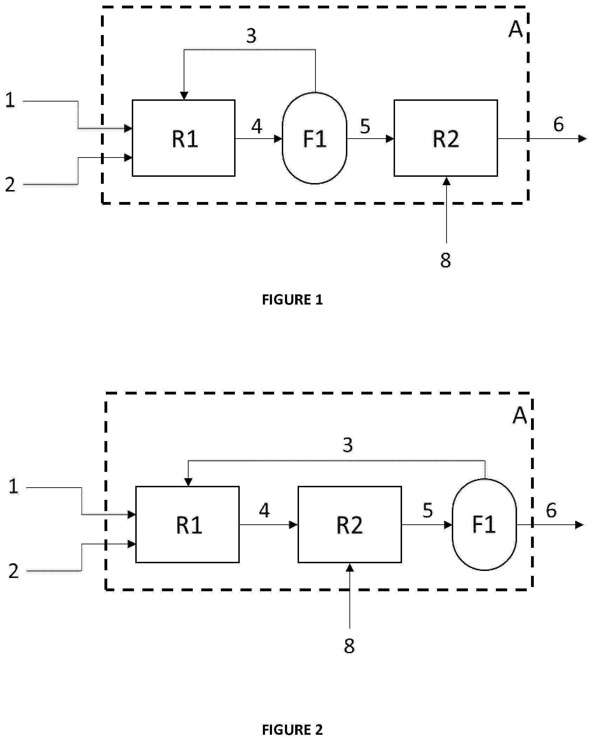

An example of such configurations is shown in figure 1, which represents a reaction zone **A** comprising a first hydrating reactor **R1,** a flasher **F1** and a second hydrating reactor **R2.** The first hydrating reactor **R1** can be fed with an initial feed of water **1** and an initial feed of alkylene oxide **2.** The reactor is operated under conditions which are adequate for the hydration of the alkaline oxide. Typical conditions for propylene oxide non catalytic hydration can be 25 bar and 125°C to 195°C (inlet - outlet temperature). The reaction product **4** exiting the first hydrating reactor **R1** is fed to a flasher **F1** were water is partially evaporated, exiting through recycle line **3** back to the first hydrating reactor **R1.** The amount of water evaporated can be controlled with the appropriate configuration of the flasher and the operation conditions; the temperature and pressure can be adjusted to provide the desired flashing temperature and flow. The bottoms of the flasher **F1,** a mixture of water and alkylene glycols, are fed as reaction product **5** into the second hydrating reactor **R2,** also fed with further alkylene oxide **8.** Before entering the second hydrating reactor **R2,** the feed **5** can be further cooled if necessary. Again, the reactor is operated under conditions which are adequate for the hydration of alkaline oxide. Typical conditions for propylene oxide non catalytic hydration can be 20 bar and 135°C to 195°C (inlet - outlet temperature). The reaction product **6** is then sent to dewatering and purification zones.

A further example of such configurations which does not form part of the presently claimed invention is shown in figure 2, which represents a reaction zone **A** comprising a first hydrating reactor **R1,** a second hydrating reactor **R2** and a flasher **F1.** The first hydrating reactor **R1** can be fed with an initial feed of water **1** and an initial feed of alkylene oxide **2.** The reactor is operated under conditions which are adequate for the hydration of the alkaline oxide. Typical conditions for propylene oxide non catalytic hydration can be 25 bar and 125°C to 195°C (inlet - outlet temperature). The reaction product **4** exiting the first hydrating reactor **R1** is fed after cooling (not shown in the figure) to the second hydrating reactor **R2,** also fed with further alkylene oxide **8.** Again, the reactor is operated under conditions which are adequate for the hydration of alkaline oxide. Typical conditions for propylene oxide non catalytic hydration can be 20 bar and 135°C to 195°C (inlet - outlet temperature). The reaction product **5** exiting the first hydrating reactor **R2** is fed to a flasher **F1** were water is partially evaporated, exiting through recycle line **3** back to the first hydrating reactor **R1.** The amount of water evaporated can be controlled with the appropriate configuration of the flasher and the operation conditions. The bottoms **6** of the flasher **F1,** a mixture of water and alkylene glycols, are then sent to dewatering and purification zones.

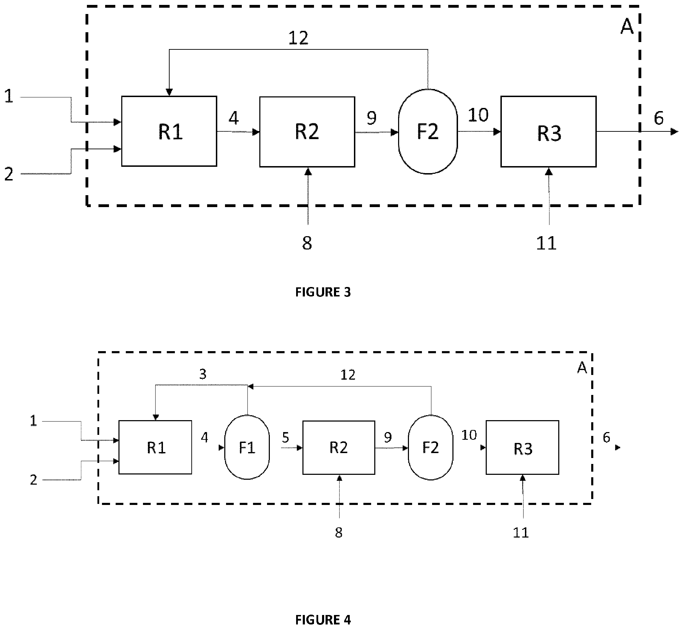

A further example is shown in figure 3. The reaction zone **A** comprises a first **R1,** a second **R2** and a third **R3** hydrating reactors, and a flasher **F2.** Thus, it differs from the configuration of figure 1 in that an additional reactor is introduced between the first hydrating reactor **R1** and the flasher. The first hydrating reactor **R1** is fed with an initial feed of water **1** and an initial feed of alkylene oxide **2.** The reactor is operated under conditions which are adequate for the hydration of the alkaline oxide. Typical conditions for propylene oxide non catalytic hydration can be 25 bar and 125°C to 195°C (inlet - outlet temperature). After undergoing a cooling process (not shown in the figure), the reaction product **4** exiting the first hydrating reactor **R1** is fed to a second hydrating reactor **R2,** being fed with alkylene oxide **8.** The reaction product **9** exiting the second hydrating reactor **R2** is fed to the flasher **F2** wherein water is partially evaporated, exiting through recycle line **12** back to the first hydrating reactor **R1.** The amount of water evaporated can be controlled with the appropriate configuration of the flasher and the operation conditions. The bottoms of the flasher **F2,** a mixture of water and alkylene glycols, are fed as reaction product **10** into the third hydrating reactor **R3,** also fed with further alkylene oxide **11.** Before entering the third hydrating reactor **R3,** the feed **10** can be further cooled if necessary. Again, the reactor is operated under conditions which are adequate for the hydration of alkaline oxide. Typical conditions for propylene oxide non catalytic hydration can be 20 bar and 135°C to 195°C (inlet - outlet temperature). The reaction product **6** is then sent to dewatering and purification zones.

A further example of a possible configuration is shown in figure 4. The reaction zone **A** comprises a first **R1,** a second **R2** and a third **R3** hydrating reactors, and a first **F1** and second flasher **F2.** The first hydrating reactor **R1** is fed with an initial feed of water **1** and an initial feed of alkylene oxide **2.** The reactor is operated under conditions which are adequate for the hydration of the alkaline oxide. Typical conditions can be for propylene oxide non catalytic hydration can be 25 bar and 125°C to 195°C (inlet - outlet temperature). The reaction product **4** exiting the first hydrating reactor **R1** is fed to a first flasher **F1** were water is partially evaporated, exiting through recycle line **3** back to the first hydrating reactor **R1.** The amount of water evaporated can be controlled with the appropriate configuration of the flasher and the operation conditions. The bottoms of the first flasher **F1,** a mixture of water and alkylene glycols, are fed as reaction product **5** to the second hydrating reactor **R2,** also fed with further alkylene oxide **8.** Again, the reactor is operated under conditions which are adequate for the hydration of alkaline oxide. Typical conditions can be for propylene oxide non catalytic hydration can be 20 bar and 135°C to 195°C (inlet - outlet temperature). The reaction product **9** exiting the second hydrating reactor **R2** is fed to the second flasher **F2** were water is partially evaporated, exiting through recycle line **12** back to the first hydrating reactor **R1.** The amount of water evaporated can be controlled with the appropriate configuration of the flasher and the operation conditions. The bottoms of the second flasher **F2,** a mixture of water and alkylene glycols, are fed as reaction product **10** to the third hydrating reactor **R3,** also fed with further alkylene oxide **11.** Again, the reactor is operated under conditions which are adequate for the hydration of alkaline oxide. Typical conditions can be for propylene oxide non catalytic hydration can be 20 bar and 135°C to 195°C (inlet - outlet temperature). The reaction product **6** is then sent to dewatering and purification zones.

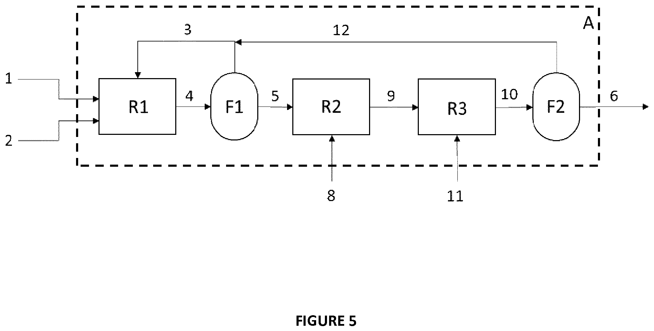

A further example of a possible configuration is shown in figure 5. The reaction zone **A** comprises a first **R1,** a second **R2** and a third **R3** hydrating reactors, and a first **F1** and second flasher **F2.** The first hydrating reactor **R1** is fed with an initial feed of water **1** and an initial feed of alkylene oxide **2.** The reactor is operated under conditions which are adequate for the hydration of the alkaline oxide. Typical conditions can be for propylene oxide non catalytic hydration can be 25 bar and 125°C to 195°C (inlet - outlet temperature). The reaction product **4** exiting the first hydrating reactor **R1** is fed to a first flasher **F1** were water is partially evaporated, exiting through recycle line **3** back to the first hydrating reactor **R1.** The amount of water evaporated can be controlled with the appropriate configuration of the flasher and the operation conditions. The bottoms of the first flasher **F1,** a mixture of water and alkylene glycols, are fed as reaction product **5** to the second hydrating reactor **R2,** also fed with further alkylene oxide **8.** Again, the reactor is operated under conditions which are adequate for the hydration of alkaline oxide. Typical conditions can be for propylene oxide non catalytic hydration can be 20 bar and 135°C to 195°C (inlet - outlet temperature). After cooling, the reaction product **9** exiting the second hydrating reactor **R2** is fed to the third hydrating reactor **R3,** also fed with further alkylene oxide **11.** Again, the reactor is operated under conditions which are adequate for the hydration of alkaline oxide. Typical conditions can be for propylene oxide non catalytic hydration can be 20 bar and 135°C to 195°C (inlet - outlet temperature). The reaction product **10** is then fed to the second flasher **F2** were water is partially evaporated, exiting through recycle line **12** back to the first hydrating reactor **R1.** The amount of water evaporated can be controlled with the appropriate configuration of the flasher and the operation conditions. The bottoms **6** of the second flasher **F2** are then sent to dewatering and purification zones.

Generally speaking, for each reactor the feeds of water (or the reaction product from the previous reactor) and of alkylene oxide can be premixed and then fed to the reactor of the reactor zone. For example, in any of the configurations exemplified by figures 1, 2, 3, 4 and 5, the feed of water **1** and of alkylene oxide **2** can be premixed and then fed to the first hydrating reactor **R1.** Also either (or both) recycle feeds **3** and **12** can be premixed with water feed **1** and/or alkylene oxide feed **2.** According to further embodiments the feeds **8** and/or **11** can be pre-mixed with the reaction products **4,** 5 or **10** before entering the corresponding hydrating reactor.

Of course, the exemplary conditions for propylene oxide non catalytic hydration given above (20-25 bar, 135-195°C) can vary depending on the alkylene oxide used, and typically ensure that the reactants are in the liquid phase and avoid a temperature overrun of this exothermic reaction. For example, the pressure can be higher in the case of ethylene oxide (it is more volatile than propylene oxide), and lower in the case of butylene oxide and other alkylene oxides having a higher boiling point.

By applying the above configurations or other possible configurations which can be devised by the skilled person within the teachings of the present disclosure less external water is required. Some advantageous alternatives discussed previously can be applied to the above configurations (not shown in the figures). For example, a first alternative is flashing the reaction product between reactors, which can partially or totally substitute the cooling means typically required to reduce the temperature between reactors. As a consequence, the energy required for inter-stage cooling is reduced. In a second alternative, the flashed water can be condensed and at the same time pre-heat the external water feed fed to the first hydrating reactor. In a third alternative, impurities which are evaporated in the flasher together with the water can be removed by means of non-condensable purge or stripper before recycling the water. Of course, in a fourth alternative the water flashed can be cooled by means of an external cooler or quench with a stream of cooler water. A combination of one or more of the above first, second, third and fourth alternatives can be installed and are within the scope of the present invention.

Therefore, the configurations exemplified above and other of the alternatives described below cannot be understood as isolated embodiments but as examples of the general disclose, and can be thus combined by the skilled person to optimize the operation of a plant within the scope of the invention.

If a flasher is placed after the last reactor before dewatering, the temperature of the crude product entering the dewatering zone is lowered and requires thus more energy consumption for dewatering. In order to improve the energy integration of the process, said crude product can be used for energy transfer operations with the products exiting any of the previous hydrating reactors. In this way, the crude product exiting the last flash is heated towards the dewatering process, while the product exiting previous reactors is cooled before entering the next reactor. In other words, before it is sent to dewatering, the crude product exiting the last flasher can be used as cooling means of at least one of the products exiting the previous hydrating reactors.

For example, the method of the invention can comprise (i) hydrating with water a first feed of alkylene oxide in a first hydrating reactor; (ii) cooling the product of said first hydrating reactor and providing a second feed of said alkylene oxide; (iii) proceeding to a second hydration in a second hydrating reactor; (iv) flash dewatering in a first flasher the product of said second hydrating reactor and recirculating into the reaction zone the water evaporated; (v) optionally, cooling the bottoms of said first flasher; (vi) mixing the bottoms of said first flasher with a third feed of said alkylene oxide; (vi) proceeding to a third hydration in a third hydrating reactor; (vii) flash dewatering in a second flasher the product of said third hydrating reactor and recirculating into the reaction zone the water evaporated; and (viii) optionally, submitting the bottoms of said second flasher to heat exchange with the product of said first hydrating reactor.

The crude product mixture exiting the reaction zone according to the present invention contains a mixture of glycols, water and other impurities, and can be further treated following known procedures. The most widespread commercial procedures send the crude product mixture directly to the dewatering zone. The most frequent scheme is the triple effect mentioned above and described in many references such as Ullmann's Encyclopedia of Industrial Chemistry, seventh Edition, Ethylene Glycol chapter, US 2012/0203015 A1 or US 5,514,388 B1. Three evaporators are arranged in series at decreasing pressures so that the vapor boiled off in one vessel can be used to heat the next due to the thermal gradient. Only the first evaporator requires an external source of heat. Vapor of the first and second evaporators is passed through the reboiler of the second and third evaporators respectively. Vapor of the last evaporator is condensed using a external cooler. Condensed water from dewatering is sent back to the reaction zone for recycling. Multiple effect evaporation does not usually remove all the water, leaving a 2 to 10 percent weight of water in the glycol stream, and is many times followed downstream by a vacuum distillation tower that completes the drying of the glycol product. Once the glycol product has been dewatered and dried, it is typically purified in a purification zone, usually through distillation.

In order to further illustrate the operation and advantages of the present invention non-limiting examples (and comparative examples) are provided.

### EXAMPLES

### Example 1 and Comparative Example 1: thermal hydration of propylene oxide (PO) using a single reactor (Example 1 is a reference example)

The operation of a single reactor thermal hydration process was simulated without flasher (Comparative Example 1, i.e. according to prior art) and with a flasher (Example 1).

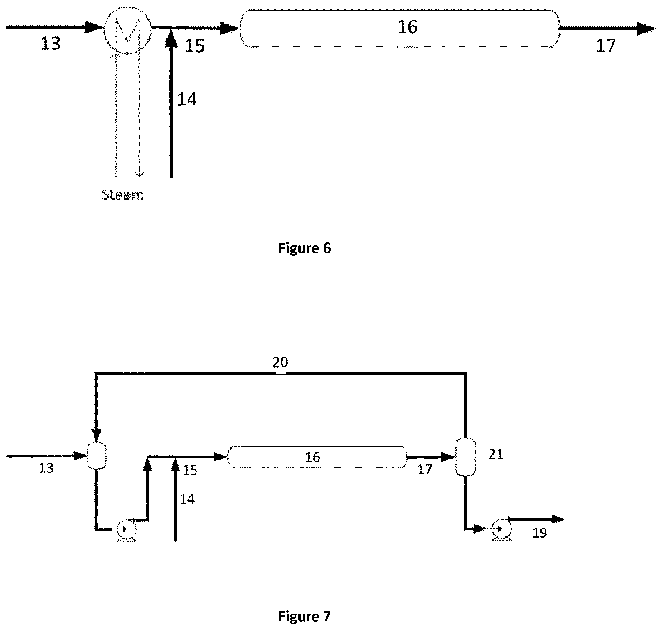

Comparative Example 1 (according to prior art) is described with reference to figure 6. A single adiabatic tubular plug-flow reactor **16** is fed with preheated water **13** (52,500 Kg/h at 83°C) mixed with PO **14** (13,125 Kg/h at 15°C) through line **15.** Thus, the molar proportion water:PO is about 9.8:1 (about 20 wt% of PO in the feed). The temperature at entry in the reactor is 120°C and it is 190°C at the exit. The product mixture exits the reactor **16** through line **17.**

Example 1 is described with reference to figure **7****,** and is similar to Comparative Example 1, but for the use of a flasher **21** and recirculation of the water **20** so evaporated (6,700 Kg/h with a composition of more than 98 wt% of water). The total water fed to the hydrating reactor **16** is therefore 57,200 Kg/h. A single adiabatic tubular plug-flow reactor **16** is fed with preheated water **13** (52,500 Kg/h at 83°C) and 6,700 Kg/h of recycled water **20** (total of 57,200 Kg/h), mixed with PO **14** (13,125 Kg/h at 15°C) through line **15.** The temperature at entry in the reactor is 128°C and it is 190°C at the exit. The product mixture exits the reactor **16** through line **17** and is sent to a drum flasher **21,** from which water is partially evaporated (6,700 Kg/h with a composition of more than 98 wt% of water) and recirculated through line **20** and mixed with water **13,** as mentioned before. Thus, the molar proportion water:PO taking into account the recirculated water is now about 11:1 (about 18 wt% of PO in the feed **15**). The bottoms of the flasher **21** are at about 150°C and are pumped through line **19** to the dewatering zone.

The glycol composition given in Kg/h obtained from the product mixture sent to dewatering in each case is summarized in Table 1:

**Table 1**

| | Comparative Example 1 | Example 1 |
|---|---|---|
| Monopropylene Glycol (MPG) | 15,884 | 16,000 |
| Dipropylene Glycol (DPG) | 1,088 | 990 |
| Tripropylene Glycol (TPG) | 40 | 33 |

In this way, the MPG output is improved by 0.7%, the DPG is reduced a 0.1% and TPG is reduced a 15%. Overall the process provides a better selectivity towards monoalkylene oxides with the same energy (about 8.96 MMKcal/h in both cases, comparing the energy used to heat stream **13,** plus the energy required to dewater stream 17 in Comparative Example 1 versus, energy needed to dewater stream 19 in Example 1).

On the other hand, the system of Example 1 can be operated so as to obtain the same glycol composition as in Comparative Example 1 (i.e. feeding in both cases the same overall amount of water to the reactor **16**). In this way, instead of improving the proportion of the monoalkylene glycol obtained, energy savings can be achieved because less water is sent to the dewatering zone. The overall effect is a reduction of the consumption to 7.5 MMKcal/h (energy required in dewatering stream **19**), a 16% reduction with respect to Comparative Example 1 (about 8.96 MMKcal/h, including the energy used to heat stream **13** plus the energy used in dewatering stream **19**), while obtaining in both cases the same amounts of MPG, DPG and TPG.

### Example 2 and Comparative Example 2: thermal hydration of propylene oxide (PO) using three adiabatic tubular plug-flow reactors

The operation of three thermal hydrating reactors was simulated without flashers (Comparative Example 2, i.e. according to prior art) and with two flashers (Example 2, i.e. according to the present invention).

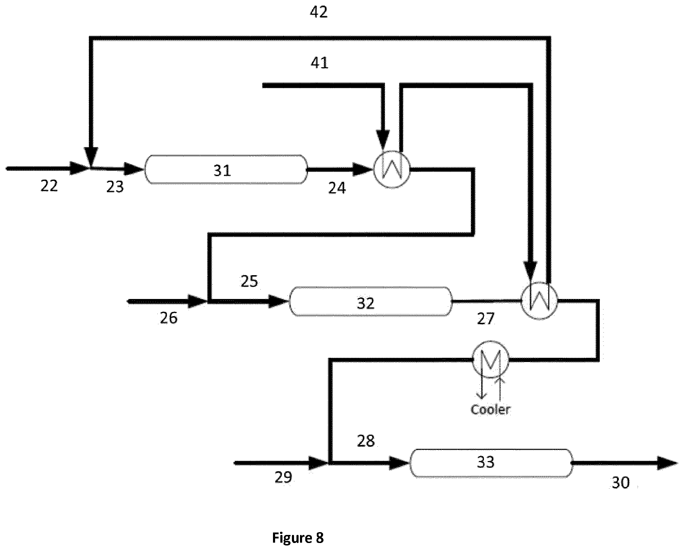

Comparative Example 2 (according to prior art) is described with reference to figure **8****.** A first adiabatic tubular plug-flow reactor **31** is fed with OP **22** (4,331 Kg/h at 15°C) and water **42** (22,900 Kg/h) to create feed **23.** The water stream **42** comes from a water stream **41** at lower temperature, which has been heated through energy exchanges with the streams (**24** and **27**) exiting the first and second adiabatic tubular plug-flow reactors (**31** and **32,** respectively). The temperature of said stream **23** when entering the first reactor **31** is 142°C and the temperature of the stream **24** exiting said first reactor **32** is 195°C. Once cooled, stream **24** is mixed with further 4,331 Kg/h of OP (**26**) at 15°C to create the feed **25** that enters the second reactor **32** and exists as stream **27** at 190°C. Said stream **27** is cooled with a cooler and mixed with further 4,331 Kg/h of OP (**29**) at 15°C to form stream **28** that enters the third reactor **33** and exits at 190°C as stream **30,** which is sent to the dewatering zone.

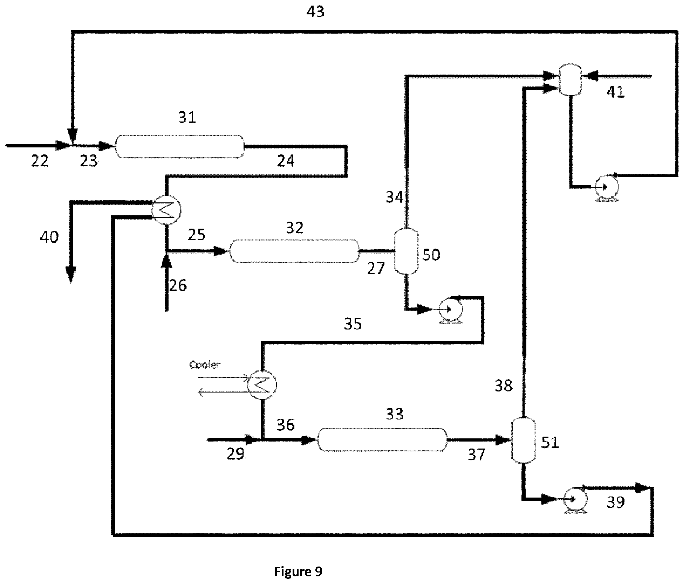

Example 2 (according to the present invention) is described with reference to figure **9****,** and is similar to Comparative Example 2 but for the inclusion of a first flasher **50** after the second reactor **32** and of a third flasher **51** after the third reactor **33.** The water evaporated in the first and second flashers (1,300 Kg/h and 2,900 Kg/h, respectively) is mixed with the water feed **41** (22,900 Kg/h) to create the water feed **43** with a total of 27,100 Kg/h.

Accordingly, a first adiabatic tubular plug-flow reactor **31** is fed with OP **22** (4,331 Kg/h at 15°C) and water **43** (27,100 Kg/h) to create stream **23.** The temperature of the stream **24** exiting said first reactor **31** is 195°C. Once cooled with the final crude product **39** (see below), stream **24** is mixed with further 4,331 Kg/h of OP (**26**) at 15°C to create the feed **25** that enters the second adiabatic tubular plug-flow reactor **32** and exists as stream **27** at 190°C. Said stream **27** is flashed in the first flasher **50** (7 bar, 1,300 Kg/h of water flashed), further cooled with a cooler and mixed with further 4,331 Kg/h of OP (**29**) at 15°C to form stream **36** that enters the third adiabatic tubular plug-flow reactor **33** and exits at 190°C as stream **37.** Stream **37** is flashed in the second flasher **51** (3 bar, 2,900 Kg/h of water flashed). As mentioned above, the final crude product **39** is used as cooling means of stream **24** so as to increase the temperature of said stream **39** before being sent to the dewatering zone.

The glycol composition given in Kg/h obtained from the product mixture sent to dewatering in each case is summarized in Table 2:

**Table 2**

| | Comparative Example 2 | Example 2 |
|---|---|---|
| Monopropylene Glycol (MPG) | 14,383 | 14,633 |
| Dipropylene Glycol (DPG) | 2,255 | 2,077 |
| Tripropylene Glycol (TPG) | 192 | 158 |

In this way, the MPG output is improved by 1.7%, the DPG is reduced a 7.9% and TPG is reduced a 17.7%. Overall the process of the invention provides a better selectivity towards monoalkylene oxides with the same energy (about 1.9 MMKcal/h in both cases, as in Comparative Example 1 and Example 1, including all energy used in the reaction zone and dewatering).

On the other hand, the system of Example 2 can be operated so as to obtain the same glycol composition as in Comparative Example 2 (i.e. feeding in both cases the same overall amount of water to the reactor **31**). In this way, instead of improving the proportion of the monoalkylene glycol obtained, energy savings can be achieved because less water is sent to the dewatering zone. The overall effect is a reduction of the consumption to 1.5 MMKcal/h, a 22% reduction with respect to Comparative Example 1 (about 1.9 MMKcal/h), while obtaining in both cases the same amounts of MPG, DPG and TPG.

## Claims

1. A method for preparing an alkylene glycol comprising hydrating with water an alkylene oxide in a hydrating reactor placed in a reaction zone, said alkylene oxide being in at least 5 wt% with respect to the total feed, **characterized in that** the product mixture of said hydrating reactor is subject to a dewatering flash process in a flasher, wherein said flasher is a flash separator drum and wherein the bottoms leaving the flasher are re-pressurized prior entry into the following hydrating reactor.

2. The method according to claim 1, wherein said flasher is operated at a pressure that ranges from reaction pressure to lower than atmospheric pressure.

3. The method according to any of claims 1 or 2, wherein said flasher is a flash separator drum for adiabatic flash vaporization without any plates.

4. The method according to to any of the previous claims, wherein the reaction zone comprises a first hydrating reactor, a second hydrating reactor and a third hydrating reactor.

5. The method according to claim 4, wherein the product of the second hydrating reactor is subject to a dewatering flash process in a first flasher and the product of the third hydrating reactor is subject to a dewatering flash process in a second flasher.

6. The method according to any of claims 4 or 5, wherein flashed water is condensed and at the same time pre-heats external water fed to the first reactor.

7. The method according to any of the previous claims, wherein the dewatering flash process takes place in a Flash unit distinct from a reactor.

8. The method according to any of claims 4 to 7 wherein each hydrating reactor comprises a feed of alkylene oxide.

9. The method according to any of the previous claims wherein the water evaporated in at least one of the flashers is returned to a previous hydrating reactor.

10. The method according to any of the previous claims wherein the product of the last hydrating reactor is subject to a dewatering flash process in a flasher.

11. The method according to any of the previous claims, comprising
(i) hydrating with water a first feed of alkylene oxide in a first hydrating reactor,
(ii) cooling the product of said first hydrating reactor and providing a second feed of said alkylene oxide;
(iii) proceeding to a second hydration in a second hydrating reactor;
(iv) flash dewatering in a first flasher the product of said second hydrating reactor and recirculating into the reaction zone the water evaporated;
(v) optionally, cooling the bottoms of said first flasher;
(vi) mixing the bottoms of said first flasher with a third feed of said alkylene oxide;
(vii) proceeding to a third hydration in a third hydrating reactor;
(viii) flash dewatering in a second flasher the product of said third hydrating reactor and recirculating into the reaction zone the water evaporated; and
(ix) optionally, submitting the bottoms of said second flasher to heat exchange with the product of said first hydrating reactor.

12. The method according to any of the previous claims, wherein the product of the last hydrating reactor is subject to a dewatering flash process in a flasher and the crude product is used to cool the product of the first hydrating reactor.

13. The method according to any of the previous claims, wherein the water evaporated in at least one of the flashers is condensed and recycled to the first hydrating reactor.

14. The method according to claim 13, wherein said water evaporated in at least one of the flashers is passed through a stripper before it is recycled to the first hydrating reactor.

15. The method according to any of the previous claims wherein the product mixture leaving the reaction zone is subject to evaporation in a dewatering zone and then purified in a purification zone.

## Patentansprüche

1. Ein Verfahren zur Herstellung von Alkylenglykol umfassend Hydratisieren eines Alkylenoxids mit Wasser in einem Hydratisierungsreaktor positioniert in einer Reaktionszone, welches Alkylenoxid in Bezug auf die Gesamtbeschickung mindestens 5 Gew.-% beträgt,
**dadurch gekennzeichnet,**
**dass** das Produktgemisch des Hydratisierungsreaktors einem Entwässerungs-Entspannungsverdampfungs-Prozess in einem Entspannungsverdampfer unterzogen wird, wobei der Entspannungsverdampfer eine Entspannungsverdampfungs-Trennungstrommel ist und wobei das den Entspannungsverdampfer verlassende Sumpfprodukt vor dem Eintritt in den folgenden Hydratisierungsreaktor erneut unter Druck gesetzt wird.

2. Verfahren nach Anspruch 1,
in welchem der Entspannungsverdampfer bei einem Druck betrieben wird, der von einem Reaktionsdruck bis unter Atmosphärendruck reicht.

3. Verfahren nach einem der Ansprüche 1 oder 2,
in welchem der Entspannungsverdampfer eine Entspannungsverdampfungs-Trennungstrommel für adiabatische Entspannungsverdampfung ohne eine Platte ist.

4. Verfahren nach einem der vorangegangenen Ansprüche,
in welchem die Reaktionszone einen ersten Hydratisierungsreaktor, einen zweiten Hydratisierungsreaktor und einen dritten Hydratisierungsreaktor umfasst.

5. Verfahren nach Anspruch 4,
in welchem das Produkt des zweiten Hydratisierungsreaktors einem Entwässerungs-Entspannungsverdampfungs-Prozess in einem ersten Entspannungsverdampfer und das Produkt des dritten Hydratisierungsreaktors einem Entwässerungs-Entspannungsverdampfungs-Prozess in einem zweiten Entspannungsverdampfer unterzogen wird.

6. Verfahren nach einem der Ansprüche 4 oder 5,
in welchem ein entspannungsverdampftes Wasser kondensiert wird und gleichzeitig externes Wasser vorwärmt, das dem ersten Reaktor zugeführt wird.

7. Verfahren nach einem der vorangegangenen Ansprüche,
in welchem der Entwässerungs-Entspannungsverdampfungs-Prozess in einer Entspannungsverdampfungseinheit erfolgt, die sich von einem Reaktor unterscheidet.

8. Verfahren nach einem der Ansprüche 4 bis 7,
in welchem jeder Hydratisierungsreaktor eine Zuführung für Alkylenoxid umfasst.

9. Verfahren nach einem der vorangegangenen Ansprüche,
in welchem das in mindestens einem der Entspannungsverdampfer verdampfte Wasser in einen voranstehenden Hydratisierungsreaktor rückgeführt wird.

10. Verfahren nach einem der vorangegangenen Ansprüche,
in welchem das Produkt des letzten Hydratisierungsreaktors einem Entwässerungs-Entspannungsverdampfungs-Prozess in einem Entspannungsverdampfer unterzogen wird.

11. Verfahren nach einem der vorangegangenen Ansprüche,
umfassend
(i) Hydratisieren mit Wasser einer ersten Beschickung von Alkylenoxid in einem ersten Hydratisierungsreaktor,
(ii) Abkühlen des Produkts des ersten Hydratisierungsreaktors und Bereitstellen einer zweiten Beschickung von Alkylenoxid,
(iii) Übergang zu einer zweiten Hydratisierung in einem zweiten Hydratisierungsreaktor,
(iv) Entspannungsverdampfungsentwässern des Produkts des zweiten Hydratisierungsreaktors in einem ersten Entspannungsverdampfer und Rückführen des verdampften Wassers in die Reaktionszone,
(v) vorzugsweise Kühlen des Sumpfprodukts des ersten Entspannungsverdampfers,
(vi) Mischen des Sumpfprodukts des ersten Entspannungsverdampfers mit einer dritten Beschickung des Alkylenoxids,
(vii) Übergang zu einer dritten Hydratisierung in einem dritten Hydratisierungsreaktor,
(viii) Entspannungsverdampfungsentwässern des Produkts des dritten Hydratisierungsreaktors in einem zweiten Entspannungsverdampfer und Rückführen des verdampften Wassers in die Reaktionszone, und
(ix) vorzugsweise Unterwerfen des Sumpfprodukts des zweiten Entspannungsverdampfers einem Wärmeaustausch mit dem Produkt des ersten Hydratisierungsreaktors.

12. Verfahren nach einem der vorangegangenen Ansprüche,
in welchem das Produkt des letzten Hydratisierungsreaktors einem Entwässerungs-Entspannungsverdampfungs-Prozess in einem Entspannungsverdampfer unterzogen und das Rohprodukt zum Kühlen des Produkts des ersten Hydratisierungsreaktors verwendet wird.

13. Verfahren nach einem der vorangegangenen Ansprüche,
in welchem das verdampfte Wasser in zumindest einem der Entspannungsverdampfer kondensiert und zu dem ersten Hydratisierungsreaktor rückgeführt wird.

14. Verfahren nach einem der vorangegangenen Ansprüche,
in welchem das in mindestens einem der Entspannungsverdampfer verdampfte Wasser durch eine Strippungseinheit geleitet wird, bevor es in den ersten Hydratisierungsreaktor rückgeführt wird.

15. Verfahren nach einem der vorangegangenen Ansprüche,
in welchem das die Reaktionszone verlassende Produktgemisch in einer Entwässerungszone einer Verdampfung unterzogen und dann in einer Reinigungszone gereinigt wird.

## Revendications

1. Méthode pour préparer un alkylène glycol, comprenant une hydratation avec de l'eau d'un oxyde d'alkylène dans un réacteur d'hydratation placé dans une zone de réaction, ledit oxyde d'alkylène représentant au moins 5 % en poids de la charge totale, **caractérisée en ce que** le mélange de produits dudit réacteur d'hydratation est soumis à un procédé d'assèchement instantané dans un évaporateur instantané, dans laquelle ledit évaporateur instantané est un tambour séparateur instantané et dans lequel les produits de fond quittant l'évaporateur instantané sont repressurisés avant l'entrée dans le réacteur d'hydratation suivant.

2. Méthode selon la revendication 1, dans laquelle ledit évaporateur instantané fonctionne sous une pression située dans la plage allant de la pression réactionnelle à une pression inférieure à la pression atmosphérique.

3. Méthode selon l'une quelconque des revendications 1 et 2, dans laquelle ledit évaporateur instantané est un tambour séparateur instantané pour évaporation instantanée adiabatique sans aucune plaque.

4. Méthode selon l'une quelconque des revendications précédentes, dans laquelle la zone de réaction comprend un premier réacteur d'hydratation, un deuxième réacteur d'hydratation et un troisième réacteur d'hydratation.

5. Méthode selon la revendication 4, dans laquelle le produit du deuxième réacteur d'hydratation est soumis à un procédé d'assèchement instantané dans un premier évaporateur instantané et le produit du troisième réacteur d'hydratation est soumis à un procédé d'assèchement instantané dans un deuxième évaporateur instantané.

6. Méthode selon l'une quelconque des revendications 4 et 5, dans laquelle l'eau issue de l'évaporation instantanée est condensée et en même temps préchauffe l'eau externe introduite dans le premier réacteur.

7. Méthode selon l'une quelconque des revendications précédentes, dans laquelle le procédé d'assèchement instantané a lieu dans une unité d'évaporation instantanée distincte d'un réacteur.

8. Méthode selon l'une quelconque des revendications 4 à 7, dans laquelle chaque réacteur d'hydratation comprend une charge d'oxyde d'alkylène.

9. Méthode selon l'une quelconque des revendications précédentes, dans laquelle l'eau évaporée dans au moins l'un des évaporateurs instantanés est renvoyée vers un réacteur d'hydratation précédent.

10. Méthode selon l'une quelconque des revendications précédentes, dans laquelle le produit du dernier réacteur d'hydratation est soumis à un procédé d'assèchement instantané dans un évaporateur instantané.

11. Méthode selon l'une quelconque des revendications précédentes, comprenant
(i) l'hydratation avec de l'eau d'une première charge d'oxyde d'alkylène dans un premier réacteur d'hydratation,
(ii) le refroidissement du produit dudit premier réacteur d'hydratation et la fourniture d'une deuxième charge dudit oxyde d'alkylène ;
(iii) la mise en oeuvre d'une deuxième hydratation dans un deuxième réacteur d'hydratation ;
(iv) l'assèchement instantané dans un premier évaporateur instantané du produit dudit deuxième réacteur d'hydratation et la remise en circulation dans la zone de réaction de l'eau évaporée ;
(v) éventuellement le refroidissement des produits de fond dudit premier évaporateur instantané ;
(vi) le mélange des produits de fond dudit premier évaporateur instantané avec une troisième charge dudit oxyde d'alkylène ;
(vii) la mise en oeuvre d'une troisième hydratation dans un troisième réacteur d'hydratation ;
(viii) l'assèchement instantané dans un deuxième évaporateur instantané du produit dudit troisième réacteur d'hydratation et la remise en circulation dans la zone de réaction de l'eau évaporée ; et
(ix) éventuellement la soumission des produits de fond dudit deuxième évaporateur instantané à un échange de chaleur avec le produit dudit premier réacteur d'hydratation.

12. Méthode selon l'une quelconque des revendications précédentes, dans laquelle le produit du dernier réacteur d'hydratation est soumis à un procédé d'assèchement instantané dans un évaporateur instantané et le produit brut est utilisé pour refroidir le produit du premier réacteur d'hydratation.

13. Méthode selon l'une quelconque des revendications précédentes, dans laquelle l'eau évaporée dans au moins un des évaporateurs instantanés est condensée et recyclée vers le premier réacteur d'hydratation.

14. Méthode selon la revendication 13, dans laquelle ladite eau évaporée dans au moins un des évaporateurs instantanés passe dans un extracteur avant d'être recyclée vers le premier réacteur d'hydratation.

15. Méthode selon l'une quelconque des revendications précédentes, dans laquelle le mélange de produits quittant la zone de réaction est soumis à une évaporation dans une zone d'assèchement et ensuite purifié dans une zone de purification.
